# EUROPEAN PATENT SPECIFICATION

(11) **EP 1 057 893 B1**
(45) Date of publication and mention of the grant of the patent: **16.05.2007**
(21) Application number: 00111015.4
(22) Date of filing: 30.05.2000
(51) Int. Cl.: C12N 15/53, C12N 9/06, C12N 1/21, C12P 13/10

(54) **Method for producing L-arginine**
Verfahren zur Herstellung von L-arginin
Procédé pour la préparation de L-arginine

(30) Priority: 03.06.1999 JP 15661699
(43) Date of publication of application: 06.12.2000
(73) Proprietor: Ajinomoto Co., Inc., Tokyo 104 (JP)
(72) Inventor: Kuwabara, Yoko, c/o Ajinomoto Co., Inc., Kawasaki-shi, Kanagawa (JP); Kurahashi, Osamu c/o Ajinomoto Co., Inc., Kawasaki-shi, Kanagawa (JP); Nakamatsu, Tsuyoshi, c/o Ajinomoto Co., Inc., Kawasaki-shi, Kanagawa (JP)
(74) Representative: HOFFMANN EITLE

(56) References cited:
- EP-A- 0 035 831
- EP-A- 0 261 627
- EP-A- 1 158 043
- FR-A- 2 575 492
- BOERMANN ET AL: "Molecular analysis of the Corynebacterium glutamicum gdh gene encoding glutamate dehydrogenase" MOLECULAR MICROBIOLOGY,GB,BLACKWELL SCIENTIFIC, OXFORD, vol. 6, no. 3, February 1992 (1992-02), pages 317-326, XP000864663 ISSN: 0950-382X
- BRAVO ET AL: "Introduction of the Escherichia coli gdhA Gene into Rhizobium phaseoli: Effect on Nitrogen Fixation" JOURNAL OF BACTERIOLOGY,US,WASHINGTON, DC, vol. 170, no. 2, February 1988 (1988-02), pages 985-988, XP000864670 ISSN: 0021-9193

### Sequence Listing

<110> Ajinomoto Co., Inc.
<120> Method for Producing L-Arginine
<130> 0P950
<140>
   <141> 2000-06-03
<150> JP 11-156616
   <151> 1999-06-03
<160> 2
<170> PatentIn Ver. 2.0
<210> 1
   <211> 25
   <212> DNA
   <213> Artificial Sequence
<220>
   <223> Description of Artificial Sequence:primer amplifying gdh gene
<400> 1
   gctagcctcg ggagctctag gagat 25
<210> 2
   <211> 25
   <212> DNA
   <213> Artificial Sequence
<220>
   <223> Description of Artificial Sequence:primer for amplifying gdh gene
<400> 2
   gatctttccc agactctggc cacgc 25

## Description

### BACKGROUND OF THE INVENTION

### of the Invention

The present invention relates to an L-arginine producing bacterium and a method for producing L-arginine. L-Arginine is an industrially useful amino acid as ingredients of liver function promoting agents, amino acid transfusions, comprehensive amino acid preparations and so forth.

### Related Art

Conventional L-arginine production by fermentation has been performed by utilizing wild-type strains of coryneform bacteria; coryneform bacteria resistant to certain agents including sulfa drugs, 2-thiazolealanine, α-amino-β-hydroxyvaleric acid and the like; coryneform bacteria exhibiting L-histidine, L-proline, L-threonine, L-isoleucine, L-methionine or L-tryptophan auxotrophy in addition to the resistance to 2-thiazolealanine (Japanese Patent Laid-open (Kokai) No. 54-44096); coryneform bacteria resistant to ketomalonic acid, fluoromalonic acid or monofluoroacetic acid (Japanese Patent Laid-open No. 57-18989); coryneform bacteria resistant to argininol (Japanese Patent Laid-open No. 62-24075); coryneform bacteria resistant to X-guanidine (X represents a derivative of fatty acid or aliphatic chain, Japanese Patent Laid-open No. 2-186995) or the like.

On the other hand, there have also been disclosed methods for increasing L-arginine producing ability by enhancing L-arginine biosynthesis enzymes utilizing recombinant DNA techniques. That is, there have been disclosed methods for producing L-arginine by utilizing a microorganism belonging to the genus *Corynebacterium* or *Brevibacterium* which is made to harbor a recombinant DNA comprising a vector DNA and a DNA fragment containing genes for acetylornithine deacetylase, N-acetylglutamic acid-γ-semialdehyde dehydrogenase, N-acetyl glutamokinase and argininosuccinase derived from a microorganism belonging to the genus *Escherichia* (Japanese Patent Publication (Kokoku) No. 5-23750).

By the way, L-glutamate dehydrogenase is an enzyme that catalyzes the reaction of reductional amination of α-ketoglutaric acid to generate L-glutamic acid. While it has been known that production amount of L-glutamic acid is increased by enhancing the activity of this enzyme (Japanese Patent Laid-open Nos. 61-268185 and 63-214189, relationship of the activity of this enzyme and L-arginine producing ability has not been known.

From the Börmann et al. article "Molecular analysis of the *Corynebacterium glutamicum* GDH gene encoding glutamate dehydrogenase", Molecular Microbiology, GB, Blackwell Scientific, Oxford, Vol. 6, Neo. 3, February 1992, pp. 317 to 326, the role of GDH in glutamate biosynthesis was known. Furthermore, the sequence of gdh from coryneform bacterium was known.

The **FR A 2 575 492** discloses micro-organisms with an enhanced intracellular GDH activity.

Further, **EP A 0 261 627** mentions that the increase of productivity of L-glutamic acid would lead to an increase of other amino acids.

From document **EP 1 158 043** , coryneform bacterium enhanced in an activity of intracellular GDH was known.

### SUMMARY OF THE INVENTION

An object of the present invention is to provide further improved method for producing L-arginine compared with conventional methods, and a microorganism used for such a method

During the process of the inventors of the present invention about breeding of coryneform bacteria producing L-glutamic acid, the inventors found that, by enhancing the activity of glutamate dehydrogenase (henceforth also referred to as "GDH") in the bacteria, not only L-glutamic acid production ability but also L-aspartic acid production ability were increased. It was considered that this might be caused by the fact that the reaction generating aspartic acid from oxaloacetic acid catalyzed by aspartate aminotransferase was coupled with the GDH reaction, and the procedure of the GDH reaction forwards the reaction catalyzed by aspartate aminotransferase. Therefore, based on this finding, they paid attention to incorporation of aspartic acid in the reaction generating argininosuccinate from citrulline in the L-arginine biosynthetic pathway, and enhanced the GDH activity of L-arginine producing bacteria. As a result, they found that L-arginine producing ability was increased by the enhancement. Thus, the present invention has been accomplished.

That is, the present invention provides the followings.
(1) A microorganism which is modified to enhance an activity of intracellular glutamate dehydrogenase and which has an increased ability to accumulate L-arginine in a medium compared with an unmodified microorganism when it is cultured in a medium wherein said microorganism is selected from the group consisting of a coryneform bacterium.
(2) The microorganism according to (1), wherein the activity of intracellular glutamate dehydrogenase is enhanced by increasing copy number of a gene which codes for the intracellular glutamate dehydrogenase.
(3) The microorganism according to (2), wherein the gene which codes for the glutamate dehydrogenase is derived from a coryneform bacterium.
(4) A method for producing L-arginine, comprising the steps of culturing the microorganism of any one of (1) to (3) in a medium to produce and accumulate L-arginine in the medium, and collecting the L-arginine from the medium.

According to the present invention, L-arginine producing ability of microorganisms having L-arginine producing ability, i.e. coryneform bacteria, can be increased.

In the present invention, the term "L-arginine producing ability" means an ability of the microorganism of the present invention to accumulate L-arginine in a medium, when it is cultured in the medium.

### PREFERRED EMBODIMENTS OF THE INVENTION

Hereafter, the present invention will be explained in detail.

### <1> Microorganism of the present invention

The microorganism of the present invention is a microorganism having an L-arginine producing ability and which is enhanced in an activity of intracellular glutamate dehydrogenase. The microorganism of the present invention is not particularly limited so long as it originally has the L-arginine producing ability or it can acquire the L-arginine producing ability when its intracellular glutamate dehydrogenase is enhanced. It is selected from coryneform bacteria.

Coryneform bacteria include those bacteria having been hitherto classified into the genus *Brevibacterium* but united into the genus *Corynebacterium* at present (*Int. J. Syst. Bacteriol*., *41*, 255 (1981)), and include bacteria belonging to the genus *Brevibacterium* closely relative to the genus *Corynebacterium*. Examples of such coryneform bacteria are listed below.
*Corynebacterium acetoacidophilum*
*Corynebacterium acetoglutamicum*
*Corynebacterium alkanolyticum*
*Corynebacterium callunae*
*Corynebacterium glutamicum*
*Corynebacterium lilium (Corynebacterium glutamicum)*
*Corynebacterium melassecola*
*Corynebacterium thermoaminogenes*
*Corynebacterium herculis*
*Brevibacterium divaricatum*
*(Corynebacterium glutamicum)*
*Brevibacterium flavum (Corynebacterium glutamicum)*
*Brevibacterium immariophilum*
*Brevibacterium lactofermentum*
*(Corynebacterium glutamicum)*
*Brevibacterium roseum*
*Brevibacterium saccharolyticum*
*Brevibacterium thiogenitalis*
*Brevibacterium album*
*Brevibacterium cerinum*
*Microbacterium ammoniaphilum*

The coryneform bacteria that have the L-arginine producing ability are not particularly limited so long as they have the L-arginine producing ability. They include, for example, wild-type strains of coryneform bacteria; coryneform bacteria resistant to certain agents including sulfa drugs, 2-thiazolealanine, α-amino-β-hydroxyvaleric acid and the like; coryneform bacteria exhibiting L-histidine, L-proline, L-threonine, L-isoleucine, L-methionine or L-tryptophan auxotrophy in addition to the resistance to 2-thiazolealanine (Japanese Patent Laid-open No. 54-44096); coryneform bacteria resistant to ketomalonic acid, fluoromalonic acid, or monofluoroacetic acid (Japanese Patent Laid-open No. 57-18989); coryneform bacteria resistant to argininol (Japanese Patent Laid-open No. 62-24075); coryneform bacteria resistant to X-guanidine (X represents a derivative of fatty acid or aliphatic chain, Japanese Patent Laid-open No. 2-186995) and so forth.

Specifically, the following bacterial strains can be exemplified.
*Brevibacterium flavum* AJ11169 (FERM P-4161)
*Brevibacterium lactofermentum* AJ12092 (FERM P-7273)
*Brevibacterium flavum* AJ11336 (FERM P-4939)
*Brevibacterium flavum* AJ11345 (FERM P-4948)
*Brevibacterium lactofermentum* AJ12430 (FERM BP-2228)

The AJ11169 strain and the AJ12092 strain are the 2-thiazolealanine resistant strains mentioned in Japanese Patent Laid-open No. 54-44096, the AJ11336 strain is the strain having argininol resistance and sulfadiazine resistance mentioned in Japanese Patent Publication No. 62-24075, the AJ11345 strain is the strain having argininol resistance, 2-thiazolealanine resistance, sulfaguanidine resistance, and exhibiting histidine auxotrophy mentioned in Japanese Patent Publication No. 62-24075, and the AJ12430 strain is the strain having octylguanidine resistance and 2-thiazolealanine resistance mentioned in Japanese Patent Laid-open No. 2-186995.

### <2> Amplification of GDH activity

In order to amplify the GDH activity in a microbial cell, a recombinant DNA can be prepared by ligating a gene fragment coding for GDH with a vector functioning in the microorganism, preferably a multicopy type vector, and introduced into the microbial cell having L-arginine producing ability to transform the cell. The copy number of the gene coding for GDH in the cell of the transformant strain is thereby increased, and as a result, the GDH activity is amplified.

As the gene coding for GDH, those derived form coryneform bacteria as well as those derived from other organisms such as *Escherichia* bacteria may be used.

The nucleotide sequence of the gene coding for GDH (ghd gene) of a coryneform bacterium has already been elucidated (*Molecular Microbiology*, *6*, (3) 317-326 (1992)). Therefore, the GDH gene can be obtained by PCR (polymerase chain reaction; see White, T.J. *et al*.; *Trends Genet*. *5*, 185 (1989)) utilizing primers produced based on the nucleotide sequence, for example, the primers represented in Sequence Listing as SEQ ID NOS: 1 and 2, and coryneform bacterium chromosomal DNA as a template. The genes coding for GDH derived from other microorganisms may be obtained similarly.

The chromosomal DNA can be prepared from a bacterium, which is a DNA donor, by the method of Saito and Miura (H. Saito and K. Miura, *Biochem. Biophys*. *Acta*, *72*, 619 (1963), Text for Bioengineering Experiments, Edited by the Society for Bioscience and Bioengineering, Japan, pp.97-98, Baifukan, 1992), for example.

The *gdh* gene amplified by PCR is preferably ligated to a vector DNA autonomously replicable in a cell of an objective microorganism such as *Escherichia coli* and/or coryneform bacteria to form a recombinant DNA. By introducing this recombinant DNA into an *Escherichia coli* cell, the subsequent procedure can be made easy. The vector autonomously replicable in *Escherichia coli* cells is preferably a plasmid vector autonomously replicable in the host cell, and examples thereof include pUC19, pUC18, pBR322, pHSG299, pHSG399, pHSG398, RSF1010 and so forth.

As the vector autonomously replicable in coryneform bacterium cells, there can be mentioned pAM330 (refer to Japanese Patent Laid-open No. 58-67699), pHM1519 (refer to Japanese Patent Laid-open No. 58-77895) and so forth. Moreover, if a DNA fragment having an ability to make a plasmid autonomously replicable in coryneform bacteria is taken out from these vectors and inserted into the aforementioned vectors for *Escherichia coli*, they can be used as a so-called shuttle vector autonomously replicable in both of *Escherichia coli* and coryneform bacteria.

Examples of such a shuttle vector include those mentioned below. There are also indicated microorganisms which harbors each vector, and accession numbers thereof at international depositories are shown in the parentheses, respectively.
pAJ655 *Escherichia coli* AJ11882 (FERM BP-136)
*Corynebacterium glutamicum* SR8201 (ATCC39135)
pAJ1844 *Escherichia coli* AJ11883(FERM BP-137)
*Corynebacterium glutamicum* SR8202 (ATCC39136)
pAJ611 *Escherichia coli* AJ11884 (FERM BP-138)
pAJ3148 *Corynebacterium glutamicum* SR8203 (ATCC39137)
pAJ440 *Bacillus subtilis* AJ11901 (FERM BP-140)
pHC4 *Escherichia coli* AJ12617 (FERM BP-3532)

These vectors can be obtained from the deposited microorganisms as follows. Microbial cells collected in their exponential growth phase are lysed by using lysozyme and SDS, and centrifuged at 30000 x g. The supernatant obtained from the lysate is added with polyethylene glycol, fractionated and purified by cesium chloride-ethidium bromide equilibrium density gradient centrifugation.

In order to prepare recombinant DNA by ligating the gene encoding GDH and a vector that can function in a cell of coryneform bacterium, the vector is digested by restriction enzyme(s) corresponding to the termini of the gene containing the gene encoding GDH. Ligation is generally performed by using a ligase such as T4 DNA ligase.

To introduce the recombinant DNA prepared as described above into a microorganism, any known transformation methods that have hitherto been reported can be employed. For instance, employable are a method of treating recipient cells with calcium chloride so as to increase the permeability of DNA, which has been reported for *Escherichia coli* K-12 (Mandel, M. and Higa, A., *J. Mol*. *Biol*., *53*, 159 (1970)), and a method of preparing competent cells from cells which are at the growth phase followed by introducing the DNA thereinto, which has been reported for *Bacillus subtilis* (Duncan, C.H., Wilson, G.A. and Young, F.E., *Gene, 1*, 153 (1977)). In addition to these, also employable is a method of making DNA-recipient cells into the protoplast or spheroplast which can easily take up recombinant DNAs followed by introducing the recombinant DNA into the cells, which is known to be applicable to *Bacillus subtilis*, actinomycetes and yeasts (Chang, S. and Choen, S.N., *Molec. Gen. Genet*., *168*, 111 (1979); Bibb, M.J., Ward, J.M. and Hopwood, O.A., *Nature, 274*, 398 (1978); Hinnen, A., Hicks, J.B. and Fink, G.R., *Proc. Natl. Sci. USA*, *75*, 1929 (1978)). The transformation of coryneform bacteria can be performed by the electric pulse method (Sugimoto *et al*., Japanese Patent Laid-open No. 2-207791).

Enhancement of the GDH activity can also be achieved by introducing multiple copies of the *gdh* gene into the chromosomal DNA of the aforementioned host. In order to introduce multiple copies of the *gdh* gene in chromosomal DNA of a microorganism, the homologous recombination is carried out using a sequence whose multiple copies exist in the chromosomal DNA as targets. As sequences whose multiple copies exist in the chromosomal DNA, repetitive DNA, inverted repeats existing at the end of a transposable element can be used. Also, as disclosed in Japanese Patent Laid-open No. 2-109985, it is possible to incorporate the *gdh* gene into transposon, and allow it to be transferred to introduce multiple copies of the *gdh* gene into the chromosomal DNA. By either method, the copy number of the *gdh* gene within cells of the transformant strain increases, and as a result, the GDH activity is enhanced.

The enhancement of the GDH activity can also be obtained by, besides being based on the aforementioned gene enhancement, modifying an expression regulatory sequence for the *gdh* gene so that the expression of the *gdh* gene should be enhanced. Specifically, it can be attained by replacing an expression regulatory sequence of the *gdh* gene on chromosomal DNA or plasmid, such as a promoter, with a stronger one (refer to Japanese Patent Laid-open No. 1-215280). For example, among the promoters that can function in coryneform bacteria, *lac* promoter, *tac* promoter and *trp* promoter of *Escherichia coli* and so forth can be mentioned as strong promoters (Y. Morinaga, M. Tsuchiya, K. Miwa and K. Sano, *J*. *Biotech*., *5*, 305-312 (1987)). Moreover, trp promoter of *Corynebacterium* bacteria is also a suitable promoter (Japanese Patent Laid-open No. 62-195294). Substitution of these promoters enhances expression of the gdh gene, and hence the GDH activity is enhanced. Enhancement of the expression regulatory sequences may be combined with the increased copy number of the gdh gene.

In the microorganism of the present invention, in addition to the enhancement of the GDH activity, activities of other enzymes catalyzing reactions of the L-arginine biosynthesis. Examples of such enzymes include acetylornithine deacetylase, N-acetylglutamic acid-γ-semialdehyde dehydrogenase, N-acetylglutamokinase, argininosuccinase, N-acetylglutamate synthase, N-acetylglutamate kinase, N-acetylglutamylphosphate reductase, acetylornithine aminotransferase, N-acetylornithinase, ornithine carbamyltransferase, argininosuccinate synthase and so forth (refer to Japanese Patent Publication Nos. 5-23750 and No. 7-28749).

The aforementioned L-arginine biosynthesis enzyme genes and the *gdh* gene may be carried by the same vector, or may be separately carried by different two or more kinds of vectors.

### <3> Production of L-arginine

L-arginine can efficiently be produced by culturing a microorganism obtained as described above in a medium to produce and accumulate L-arginine in the culture, and collecting the L-amino acids from the culture.

The medium used for culture may be a well-known medium conventionally used for the production of amino acids by fermentation. That is, it may be a usual medium that contains a carbon source, nitrogen source, inorganic ions, and other organic components as required.

As the carbon source, it is possible to use sugars such as glucose, sucrose, lactose, galactose, fructose and starch hydrolysates; alcohols such as glycerol and sorbitol; or organic acids such as fumaric acid, citric acid and succinic acid and so forth.

As the nitrogen source, it is possible to use inorganic ammonium salts such as ammonium sulfate, ammonium chloride and ammonium phosphate, organic nitrogen such as soybean hydrolysates, ammonia gas, aqueous ammonia and so forth.

As trace amount organic nutrients, the medium preferably contains a suitable amount of required substance such as vitamin B₁ and L-homoserine, yeast extract and so forth. Other than those substances, a small amount of potassium phosphate, magnesium sulfate, iron ions, manganese ions and so forth may be added to the medium.

The cultivation is preferably performed under an aerobic condition for 1-7 days. Cultivation temperature is preferably 24-37°C, and pH of the medium during the cultivation is preferably 5-9. Inorganic or organic acidic or alkaline substances, ammonia gas and so forth may be used for adjusting pH. L-Arginine can usually be recovered from the fermentation medium by a combination of known techniques such as ion exchange resin method and others.

### EXAMPLES

Hereafter, the present invention will be explained more specifically with reference to the following examples.

### <1> Cloning of gdh gene derived from coryneform bacterium and production of plasmid for introduction of gdh gene

The primers shown as SEQ ID NOS: 1 and 2 were prepared based on the known *gdh* gene sequence of *Corynebacterium glutamicum* (*Molecular Microbiology*, *6*, (3) 317-326 (1992)), and PCR was performed by using chromosomal DNA of *Brevibacterium lactofermentum* ATCC13869 as a template to obtain a *gdh* gene fragment. DNA was synthesized in a conventional manner according to the phosphoamidite method (refer to *Tetrahedron Letters* (1981), *22*, 1859)) using a DNA synthesizer Model 380B produced by Applied Biosystems. The PCR was performed by repeating a cycle consisting of reactions at 94°C for 1 minute, 55°C for 1 second and 72°C for 2.5 minutes for 25 times. The amplified DNA fragment was cloned into the SmaI site in the multi-cloning site of the cloning vector pHSG399 (produced by Takara Shuzo) to prepare pHSG-GDH.

Then, in order to make pHSG-GDH autonomously replicable in coryneform bacteria, a replication origin (Japanese Patent Laid-open No. 5-7491) of the already obtained plasmid pHM1519 autonomously replicable in coryneform bacteria (Miwa, K. *et al., Agric. Biol. Chem*., *48*, 2901-2903 (1984), Japanese Patent Laid-open No. 58-192900) was introduced. Specifically, pHM1519 was digested with restriction enzymes *Bam*HI and *Kpn*I to obtain a gene fragment containing a replication origin, and the obtained fragment was blunt-ended by using Blunting kit produced by Takara Shuzo, and then inserted into the *Sal*I site of pHSG-GDH using a *Sal*I linker (produced by Takara Shuzo) to prepare pGDH. pHM1519 can be prepared from *Corynebacterium glutamicum* ATCC13058.

### <2> Preparation of L-arginine producing strain of coryneform bacterium transformed with gdh gene

pGDH was introduced into an L-arginine producing bacterium, *Brevibacterium flavum* AJ11345 strain. The introduction of the plasmid was performed by using the electric pulse method (refer to Japanese Patent Laid-open No. 2-207791). The transformant was selected as a chloramphenicol resistant strain on a CM2G plate medium (containing 10 g of polypeptone, 10 g of yeast extract, 5 g of glucose, 5 g of NaCl, and 15 g of agar in 1 L of pure water, pH 7.2) containing 4 µg/ml of chloramphenicol to obtain AJ11345/pGDH.

The *Brevibacterium flavum* AJ11345 was deposited at National Institute of Bioscience and Human-Technology, Agency of Industrial Science and Technology, Ministry of International Trade and Industry (postal code 305-8566, 1-3 Higashi 1-chome, Tsukuba-shi, Ibaraki-ken, Japan) on April 25, 1979 and received an accession number of FERM P-4948, and transferred from the original deposit to international deposit based on Budapest Treaty on September 27, 1999, and has been deposited as deposition number of FERM BP-6894.

### <3> Production of L-arginine

AJ11345 and AJ11345/pGDH were each plated on an agar medium containing 0.5 g/dl of glucose, 1 g/dl of polypeptone, 1 g/dl of yeast extract, 0.5 g/dl of NaCl, and 5 µg/l of chloramphenicol, and cultured at 31.5°C for 20 hours. One platinum loop of the obtained bacterial cells were inoculated into a medium containing 4 g/dl of glucose, 6.5 g/dl of ammonium sulfate, 0.1 g/dl of KH₂PO₄, 0.04 g/dl of MgSO₄, 0.001 g/dl of FeSO₄, 0.01 g/dl of MnSO₄, 5 µg/dl of vitamin B₁, 5 µg/dl of biotin and soybean hydrolysate (45 mg/dl in terms of the amount of N), and cultured at 31.5°C for 50 hours in a flask with shaking. Production amounts of α-ketoglutaric acid, L-arginine and L-aspartic acid are shown in Table 1 for each strain.

**Table 1**

| Strain/Plasmid | α-Ketoglutaric acid | | L-Arginine | | L-Aspartic acid | |
|---|---|---|---|---|---|---|
| | (g/L) | (mol/L) | (g/L) | (mol/L) | (g/L) | (mol/L) |
| AJ11345 | 0.30 | 0.002 | 13.30 | 0.076 | 0.00 | 0.00 |
| AJ11345/pGDH | 0.06 | 0.0004 | 14.00 | 0.080 | 0.20 | 0.002 |

The results of Table 1 show that the production amount of L-arginine was more increased compared with the decrease of α-ketoglutaric acid, when the GDH activity of the L-arginine producing bacterium was enhanced. Moreover, the strain of which GDH activity was enhanced showed an increased byproduction amount of L-aspartic acid. This indicates that the increase of the L-arginine production amount was not due to only the decrease of byproduction amount of α-ketoglutaric acid and the increase of L-glutamic acid, which was a precursor of L-arginine. It has been known that, in the L-arginine biosynthesis system, L-aspartic acid is incorporated as a substrate in the reaction catalyzed by argininosuccinate synthase, and it is considered that the increase in supply of aspartic acid by GDH promoted this reaction, and thereby increased the production amount of L-arginine. That is, the enhancement of the GDH activity increased both of the supplied amounts of L-glutamic acid and L-aspartic acid (refer to Reference Example), and thus the L-arginine producing ability was markedly increased.

### Reference Example

pGDH was introduced into an L-glutamic acid producing bacterium, Brevibacterium lactofermentum AJ13029 strain. The strain AJ13029 was deposited at National Institute of Bioscience and Human-Technology, Agency of Industrial Science and Technology, Ministry of International Trade and Industry (postal code 305-8566, 1-3 Higashi 1-chome, Tsukuba-shi, Ibaraki-ken, Japan) on September 2, 1994 and received an accession number of FERM P-14501, and transferred from the original deposit to international deposit based on Budapest Treaty on August 1, 1995, and has been deposited as deposition number of FERM BP-5189.

The introduction of the plasmid was performed by using the electric pulse method (refer to Japanese Patent Laid-open No. 2-207791). The transformant was selected as a chloramphenicol resistant strain on a CM2G plate medium (containing 10 g of polypeptone, 10 g of yeast extract, 5 g of glucose, 5 g of NaCl, and 15 g of agar in 1 L of pure water, pH 7.2) containing 4 µg/ml of chloramphenicol to obtain AJ13029/pGDH.

AJ13029 and AJ13029/pGDH were inoculated into a medium containing 60g of glucose, 2g of KH₂PO₄, 1.5g of MgSO₄·7H₂O, 15mg of FeSO₄·7H₂O, 5mg of MnSO₄·4H₂O, 50ml of soybean hydrolysate, 2mg of biotin, 3mg of thiamine hydrochloride in 1 L of water, pH 8.0, and cultured at 31.5°C for until the sugar contained in the culture medium was consumed. Obtained culture were inoculated at the concentration of 5% into the same medium as described above and cultured at 37°C for until the sugar contained in the culture medium was consumed. Production amounts of L-glutaminc acid and L-aspartic acid are shown in Table 2 for each strain.

**Table 2**

| Strain/ Plasmid | L-Glutamic Acid | | L-Aspartic acid | |
|---|---|---|---|---|
| | (g/L) | (mol/L) | (g/L) | (mol/L) |
| AJ13029 | 33 | 0.22 | 0.49 | 0.0037 |
| AJ13029/pGDH | 35 | 0.24 | 0.95 | 0.0071 |

## Claims

1. A microorganism which is modified to enhance an activity of intracellular glutamate dehydrogenase and which has an increased ability to accumulate L-arginine in a medium compared with an unmodified microorganism when it is cultured in the medium, wherein said microorganism is selected from a group consisting of coryneform bacteria.

2. The microorganism according to claim 1, wherein the activity of intracellular glutamate dehydrogenase is enhanced by increasing copy number of a gene which codes for the intracellular glutamate dehydrogenase.

3. The microorganism according to claim 2, wherein the gene which codes for the glutamate dehydrogenase is derived from a coryneform bacterium.

4. A method for producing L-arginine, comprising the steps of:
culturing the microorganism of any one of claims 1 to 3 in a medium to produce and accumulate L-arginine in the medium; and
collecting the L-arginine from the medium.

## Patentansprüche

1. Mikroorganismus, der modifiziert ist, um eine Aktivität der intrazellulären Glutamatdehydrogenase zu verstärken und der eine erhöhte Fähigkeit zur Anhäufung von L-Arginin in einem Medium im Vergleich mit einem nichtmodifizierten Mikroorganismus, wenn er in dem Medium kultiviert wird, aufweist, wobei der Mikroorganismus gewählt ist aus der Gruppe, bestehend aus coryneformen Bakterien.

2. Mikroorganismus gemäß Anspruch 1, wobei die Aktivität der intrazellulären Glutamatdehydrogenase durch Erhöhung der Kopiezahl eines Gens verstärkt wird, das die intrazelluläre Glutamatdehydrogenase kodiert.

3. Mikroorganismus gemäß Anspruch 2, wobei das Gen, das die Glutamatdehydrogenase kodiert, von einem coryneformen Bakterium abstammt.

4. Verfahren zur Erzeugung von L-Arginin, umfassend die folgenden Schritte:
Kultivierung des Mikroorganismus gemäß einem der Ansprüche 1 bis 3 in einem Medium zur Erzeugung und Anhäufung von L-Arginin in dem Medium; und
Sammeln des L-Arginins aus dem Medium.

## Revendications

1. Microorganisme qui est modifié pour améliorer une activité de glutamate déshydrogénase intracellulaire et qui présente une capacité accrue d'accumuler la L-arginine dans un milieu par rapport à un microorganisme non modifié lorsqu'il est mis en culture dans le milieu, ledit microorganisme étant choisi dans un groupe constitué des bactéries coryneformes.

2. Microorganisme selon la revendication 1, dans lequel l'activité de glutamate déshydrogénase intracellulaire est améliorée en augmentant le nombre de copies d'un gène qui code pour la glutamate déshydrogénase intracellulaire.

3. Microorganisme selon la revendication 2, dans lequel le gène qui code pour la glutamate déshydrogénase est dérivé d'une bactérie coryneforme.

4. Procédé pour produire la L-arginine, comprenant les étapes consistant à :
mettre en culture le microorganisme de l'une quelconque des revendications 1 à 3 dans un milieu pour produire et accumuler la L-arginine dans le milieu ; et
recueillir la L-arginine du milieu.
